# EUROPEAN PATENT APPLICATION

(11) **EP 4 640 174 A1**
(43) Date of publication of application: **29.10.2025**
(21) Application number: 25172197.3
(22) Date of filing: 24.04.2025
(51) Int. Cl.: A61B 18/14, A61B 17/00, A61B 18/00

(54) **ELECTROSURGICAL LEAFLET LACERATION DEVICE**

(30) Priority: 24.04.2024 US 202463637899 P; 04.04.2025 US 202519170545
(71) Applicant: Medtronic, Inc., Minneapolis, Minnesota 55432 (US)
(72) Inventor: Horrigan, John B., Minneapolis, 55432 (US)
(74) Representative: Zimmermann & Partner Patentanwälte mbB

(57) **Abstract**

An electrosurgical leaflet laceration device is configured to transition from a delivery configuration to a deflected configuration. The electrosurgical leaflet laceration device includes a handle, a catheter shaft extending distally from the handle and including an electrosurgical wire lumen and a side opening, and an electrosurgical wire disposed within the electrosurgical wire lumen of the catheter shaft. The side opening of the catheter shaft is configured to expose a cutting segment of the electrosurgical wire with the electrosurgical leaflet laceration device in the deflected configuration. The cutting segment of the electrosurgical wire is configured to be energized cut tissue abutting or in contact with the cutting segment.

## Description

### CROSS-REFERENCE TO RELATED APPLICATIONS

This application claims the benefit of U.S. Provisional Patent Application Serial No. 63/637,899, filed April 24, 2024, and U.S. Patent Application Serial No. 19/170,545, filed April 4, 2025, the entire contents of which are incorporated herein by reference.

### FIELD

The present application is related to valve leaflet laceration devices, and more specifically to transcatheter leaflet laceration devices.

### BACKGROUND

The human heart is a four chambered, muscular organ that provides blood circulation through the body during a cardiac cycle. The four main chambers include the right atrium and right ventricle which supplies the pulmonary circulation, and the left atrium and left ventricle which supplies oxygenated blood received from the lungs to the remaining body. To ensure that blood flows in one direction through the heart, atrioventricular valves (tricuspid and mitral valves) are present between the junctions of the atria and the ventricles, and semi-lunar valves (pulmonary valve and aortic valve) govern the exits of the ventricles leading to the lungs and the rest of the body. These valves contain leaflets or cusps that open and shut in response to blood pressure changes caused by the contraction and relaxation of the heart chambers. The leaflets move apart from each other to open and allow blood to flow downstream of the valve, and coapt to close and prevent backflow or regurgitation in an upstream manner.

Diseases associated with heart valves, such as those caused by damage or a defect, can include stenosis and valvular insufficiency or regurgitation. For example, valvular stenosis causes the valve to become narrowed and hardened which can prevent blood flow to a downstream heart chamber from occurring at the proper flow rate and may cause the heart to work harder to pump the blood through the diseased valve. Valvular insufficiency or regurgitation occurs when the valve does not close completely, allowing blood to flow backwards, thereby causing the heart to be less efficient. A diseased or damaged valve, which can be congenital, age-related, drug-induced, or in some instances, caused by infection, can result in an enlarged, thickened heart that loses elasticity and efficiency. Some symptoms of heart valve diseases can include weakness, shortness of breath, dizziness, fainting, palpitations, anemia and edema, and blood clots, which can increase the likelihood of stroke or pulmonary embolism. Symptoms can often be severe enough to be debilitating and/or life threatening.

Heart valve prostheses have been developed for repair and replacement of diseased and/or damaged heart valves. Such heart valve prostheses can be percutaneously delivered and deployed at the site of the diseased heart valve through catheter-based systems. Heart valve prostheses can be delivered while in a low profile or compressed/collapsed arrangement so that the heart valve prosthesis can be advanced through the patient's vasculature. Once positioned at the treatment site, the heart valve prosthesis can be expanded to engage tissue at the diseased heart valve region to, for instance, hold the heart valve prosthesis in position.

For the replacement of an aortic valve, transaortic valve replacement (TAVR) is an option for the treatment of patients. With a transcatheter replacement of an aortic valve, there is a risk of coronary obstruction related to the valve leaflets of the failed heart valve preventing flow of blood to the coronary arteries. To overcome this challenge, procedures have been developed to lacerate one or more of the valve leaflets to prevent coronary obstruction. One such procedure for lacerating a valve leaflet is the bioprosthetic aortic scallop intentional laceration to prevent iatrogenic coronary artery obstruction (BASILICA) procedure. The BASILICA procedure requires the perforation of the valve leaflet with a guidewire, grasping and externalizing a distal portion of the wire beneath the valve leaflet, and electrifying the wire to lacerate the valve leaflet. This procedure is complex, technically challenging, lengthy, and requires two operators. Accordingly, there remains a need for improved devices and methods to lacerate heart valve leaflets.

### BRIEF SUMMARY

The techniques and devices of this disclosure generally relate to electrosurgical leaflet laceration device and methods for lacerating valve leaflets.

In an example hereof, an electrosurgical leaflet laceration device having a delivery configuration and a deflected configuration includes: a handle; a catheter shaft extending distally from the handle, the catheter shaft including an electrosurgical wire lumen and a side opening; and an electrosurgical wire disposed within the electrosurgical wire lumen of the catheter shaft, the electrosurgical wire having a cutting segment. The side opening of the catheter shaft is configured to expose the cutting segment of the electrosurgical wire with the electrosurgical leaflet laceration device in the deflected configuration. The cutting segment of the electrosurgical wire is configured to be energized cut tissue abutting or in contact with the cutting segment.

In another example hereof, in the electrosurgical leaflet laceration device of any of the preceding or following examples, the electrosurgical wire is configured to transition the electrosurgical leaflet laceration device between the delivery configuration and the deflected configuration.

In another example hereof, in the electrosurgical leaflet laceration device of any of the preceding or following examples, a proximal portion of the electrosurgical wire is coupled to an actuator in the handle and a distal portion of the electrosurgical wire is fixedly coupled to a distal end of the catheter shaft, and the actuator is configured to apply or release tension on the electrosurgical wire to apply or release a proximal force on the distal end of the catheter shaft to bend the catheter shaft to transition the electrosurgical leaflet laceration device between the delivery configuration and the deflected configuration.

In another example hereof, in the electrosurgical leaflet laceration device of any of the preceding or following examples, the catheter shaft includes a proximal portion, a distal portion, and a deflection zone portion disposed between the proximal portion and the distal portion, wherein the deflection zone portion is more flexible than the proximal portion and the distal portion such that the catheter shaft is configured to bend or deflect at the deflection zone portion to transition the electrosurgical leaflet laceration device from the delivery configuration to the deflected configuration.

In another example hereof, in the electrosurgical leaflet laceration device of any of the preceding or following examples, the side opening is disposed within the deflection zone portion of the catheter shaft.

In another example hereof, the electrosurgical leaflet laceration device of any of the preceding or following examples further includes a balloon on a distal portion of the catheter shaft, the balloon having an uninflated state and an inflated state, wherein the balloon is configured to splay or open a perforation in a valve leaflet with the balloon in the inflated state.

In another example hereof, in the electrosurgical leaflet laceration device of any of the preceding or following examples, the electrosurgical wire includes an insulative covering, and wherein the cutting segment is non-insulated.

In another example hereof, a metho of lacerating a valve leaflet comprises: advancing an electrosurgical leaflet laceration device to a treatment site adjacent a valve leaflet; positioning a distal tip of a catheter shaft of the electrosurgical leaflet laceration device adjacent a base of the valve leaflet; distally advancing a guidewire through the catheter shaft and through the valve leaflet to puncture the valve leaflet to form a puncture hole; distally advancing the electrosurgical leaflet laceration device through the puncture hole in the valve leaflet; positioning a side opening of the catheter shaft within the puncture hole in the valve leaflet and adjacent to tissue of the valve leaflet; proximally retracting an electrosurgical wire of the electrosurgical leaflet laceration device to transition the electrosurgical leaflet laceration device from a delivery configuration to a deflected configuration in which the catheter shaft is bent adjacent the side opening and a cutting segment of the electrosurgical wire is longitudinally aligned with the side opening; energizing the electrosurgical wire; proximally retracting the electrosurgical leaflet laceration device in the deflected configuration such that the electrosurgical wire lacerates the valve leaflet; and deenergizing the electrosurgical wire.

In another example hereof, the method of any of the preceding or following examples further includes, after distally advancing the electrosurgical leaflet laceration device through the puncture hole in the valve leaflet and prior to positioning the side opening within the puncture hole, inflating a balloon of the electrosurgical leaflet laceration device to enlarge the puncture hole.

In another example hereof, the method of any of the preceding or following examples further includes, after deenergizing the electrosurgical wire, proximally retracting the electrosurgical leaflet laceration device to remove the electrosurgical leaflet laceration from the treatment site.

In another example hereof, in the method of any of the preceding or following examples, the valve leaflet is a native heart valve leaflet.

In another example hereof, in the method of any of the preceding or following examples, the native heart valve leaflet is a native aortic valve leaflet.

In another example hereof, in the method of any of the preceding or following examples, the valve leaflet is a prosthetic valve leaflet of a previously implanted heart valve prosthesis.

In another example hereof, in the method of any of the preceding or following examples, proximally retracting the electrosurgical wire comprises actuating an actuator of the electrosurgical leaflet laceration device to apply a proximal force on a proximal portion of the electrosurgical wire coupled to the actuator, wherein a distal portion of the electrosurgical wire is fixedly coupled to a distal portion of the catheter shaft such that the proximal force causes the catheter shaft to bend.

In another example hereof, in the method of any of the preceding or following examples, the actuator comprises a first contact and the electrosurgical leaflet laceration device further comprises a second contact, wherein the first contact and the second contact must be in contact to energize the electrosurgical wire.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

The foregoing and other features and advantages of the present disclosure will be apparent from the following description of embodiments hereof as illustrated in the accompanying drawings. The accompanying drawings, which are incorporated herein and form a part of the specification, further serve to explain the principles of the present disclosure and to enable a person skilled in the pertinent art to make and use the embodiments of the present disclosure. The drawings are not to scale.
FIG. 1 depicts a side view of an electrosurgical leaflet laceration device according to embodiments hereof.
FIG. 2 depicts a side cross-sectional view of the electrosurgical leaflet laceration device of FIG. 1.
FIG. 3 depicts a cross-sectional view taken along line A-A of FIG. 1.
FIG. 4 depicts a side view of an electrosurgical wire of the electrosurgical leaflet laceration device of FIG. 1 according to embodiments hereof.
FIG. 5A depicts a side view of the electrosurgical leaflet laceration device of FIG. 1 in a deflected configuration.
FIG. 5B depicts a close-up view of an embodiment of the handle of an embodiment of the electrosurgical leaflet laceration device of FIG. 1 with the connector and actuator acting as a
FIG. 6 is a flow chart depicting a method for lacerating a valve leaflet with the electrosurgical leaflet laceration device of FIG. 1, according to embodiments hereof.
FIGS. 7-14 depict illustrations of several of the steps of the method of FIG. 6 for lacerating a valve leaflet with the electrosurgical leaflet laceration device of FIG. 1.
FIG. 15 depicts a side view of an electrosurgical leaflet laceration device according to embodiments hereof.
FIG. 16 depicts a side cross-sectional view of the electrosurgical leaflet laceration device of FIG. 15.
FIG. 17 depicts a cross-sectional view taken along line A-A of FIG. 15.
FIG. 18 depicts a side view of an electrosurgical wire of the electrosurgical leaflet laceration device of FIG. 15 according to embodiments hereof.
FIG. 19 depicts a side view of the electrosurgical leaflet laceration device of FIG. 1 in a deflected configuration.
FIG. 20 is a flow chart depicting a method for lacerating a valve leaflet with the electrosurgical leaflet laceration device of FIG. 15, according to embodiments hereof.
FIGS. 21-26 depict illustrations of several of the steps of the method of FIG. 20 for lacerating a valve leaflet with the electrosurgical leaflet laceration device of FIG. 15.

### DETAILED DESCRIPTION OF THE INVENTION

Specific embodiments of the present disclosure are now described with reference to the figures wherein like reference numbers indicate identical or functionally similar elements. The following detailed description describes examples of embodiments of the invention and is not intended to limit the present technology or the application and uses of the present technology. Although the description of embodiments hereof is in the context of a native aortic heart valve, the present technology may also be used in other valve locations. For example, embodiments of electrosurgical leaflet laceration devices described herein may be utilized with a pulmonary, aortic, mitral, or tricuspid valve, or may be utilized with a valve prosthesis configured for placement within a venous valve or within other body passageways where it is deemed useful. Furthermore, there is no intention to be bound by any expressed or implied theory presented in the preceding Field, Background, Brief Summary, or the following Detailed Description.

The terms "distal" and "proximal," when used in the following description to refer to a native vessel, native valve, or a device to be implanted into a native vessel or native valve, such as a heart valve prosthesis, are with reference to the direction of blood flow. Thus, "distal" and "distally" refer to positions in a downstream direction with respect to the direction of blood flow, and the terms "proximal" and "proximally" refer to positions in an upstream direction with respect to the direction of blood flow. When used in the following description to refer to a catheter or similar device (such as an electrosurgical leaflet laceration device) "distal" and "proximal" are with respect to the location of an operator using the device or the handle of such a device. Thus, distal" and "distally" refer to positions in a direction away from the operator or handle end of the device, and the terms "proximal" and "proximally" refer to positions in a direction toward the operator or handle end of the device.

Embodiments disclosed herein are directed to electrosurgical leaflet laceration devices configured to lacerate a valve leaflet. The electrosurgical leaflet laceration devices include a deflectable catheter shaft with a side opening on a distal portion thereof and an electrosurgical wire. The side opening exposes a portion of the electrosurgical wire. The catheter shaft is advanced through a perforation at or near a base or nadir of a valve leaflet. The catheter shaft is deflected, creating a bend at the side opening and exposing the electrosurgical wire to the adjacent valve leaflet tissue. The electrosurgical wire is energized, and the catheter shaft is proximally retracted to lacerate tissue of the valve leaflet.

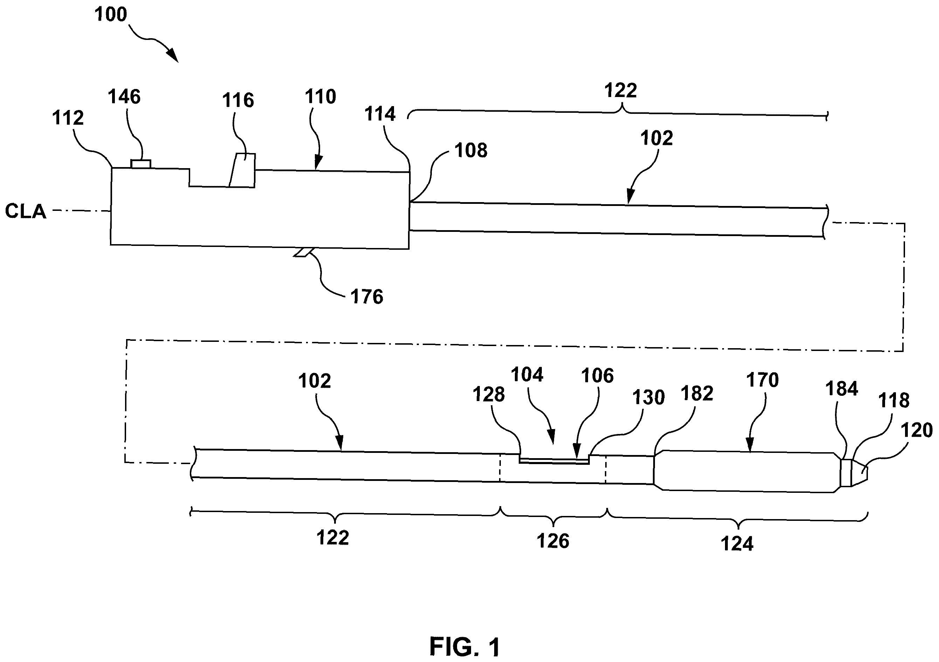

FIGS. 1-5B illustrate an electrosurgical leaflet laceration device 100 according to embodiments hereof. The electrosurgical leaflet laceration device 100 includes a handle 110, a catheter shaft 102 having a side opening 104, an electrosurgical wire 106, and a balloon 170. The electrosurgical leaflet laceration device 100 includes a delivery configuration for delivery of the electrosurgical leaflet laceration device 100 to a desired treatment location, and a deflected configuration for lacerating a valve leaflet. One skilled in the art will realize that FIGS. 1-5 illustrate one example of an electrosurgical leaflet laceration device and that existing components illustrated in FIGS. 1-5 may be removed and/or additional components may be added to the electrosurgical leaflet laceration device 100.

The electrosurgical leaflet laceration device 100 is configured to cut or lacerate a valve leaflet of a heart valve when in the deflected configuration. In the embodiment shown in of FIGS. 1-5, the electrosurgical leaflet laceration device 100 is configured to lacerate a leaflet of a native aortic valve in preparation for a transcatheter aortic valve implantation (TAVI) or of a prosthetic leaflet from a previously implanted prosthetic heart valve in preparation for a valve-in-valve transcatheter aortic valve replacement (TAVR). Such laceration of native leaflets or prosthetic leaflets functions to reduce or eliminate coronary artery obstruction by the native or prosthetic leaflets after the TAVI or the valve-in-valve TAVR.

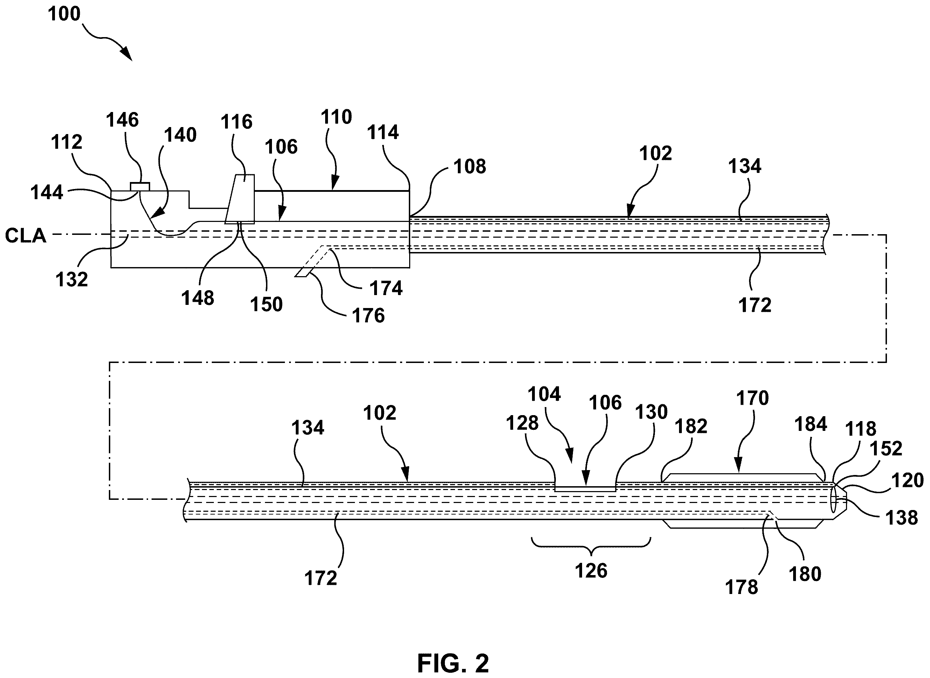

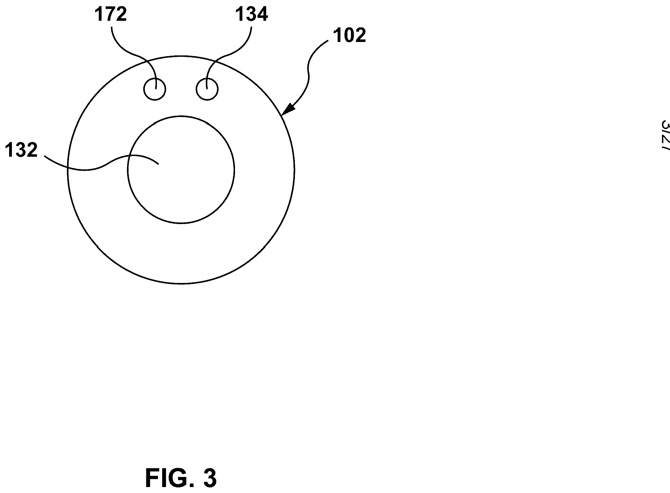

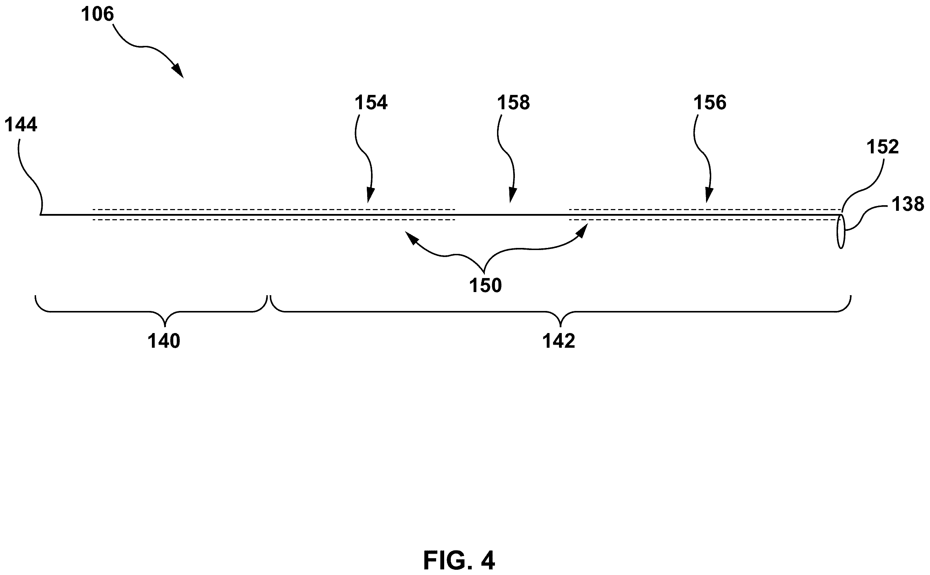

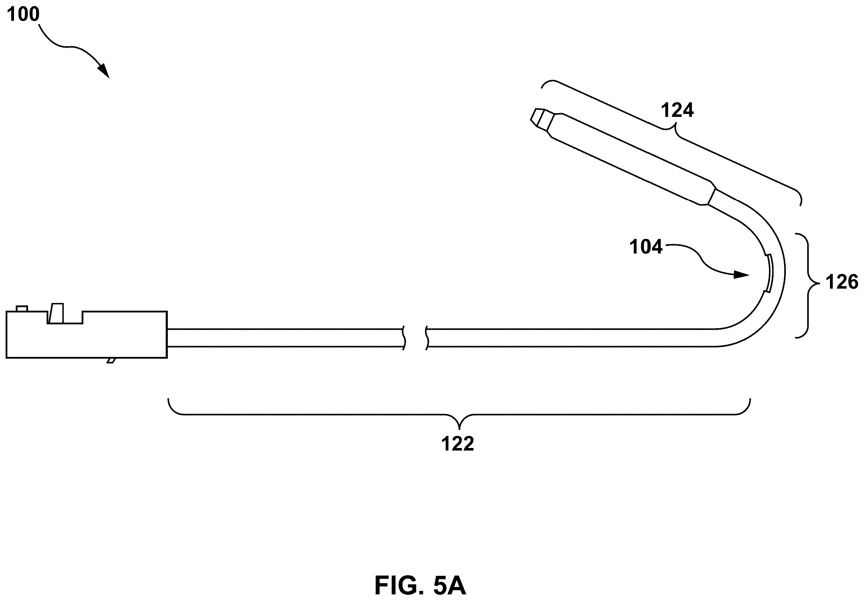

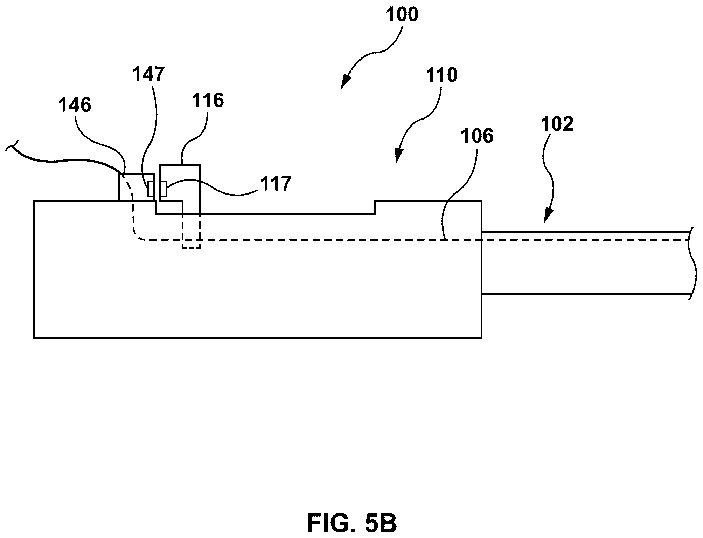

FIG. 1 is a side view of the electrosurgical leaflet laceration device 100 in a delivery configuration. FIG. 2 is a side cross sectional view of the electrosurgical leaflet laceration device 100. FIG. 3 is a cross-sectional view of the electrosurgical leaflet laceration device 100 taken along line A-A of FIG. 1. FIG. 4 is a side view of an electrosurgical wire 106 of the electrosurgical leaflet laceration device 100. FIG. 5 is a side view of the electrosurgical leaflet laceration device 100 in a deflected configuration.

As shown in FIGS. 1-3, the handle 110 enables a clinician to manipulate a distal portion of the electrosurgical leaflet laceration device 100 and may include actuators for moving parts of the device relative to other parts. The handle 110 includes a proximal end 112 and a distal end 114. In embodiments herein, the handle 110 includes an actuator 116 configured to apply or release tension on the electrosurgical wire 106 to transition the electrosurgical leaflet laceration device 100 between the delivery configuration and the deflected configuration. The handle 110 may have any shape or size appropriate for convenient handling and manipulation by a user. The actuator 116 may have any shape, size, or movement suitable for the purposes described herein.

The catheter shaft 102 includes a proximal end 108 coupled to the handle 110 and distally extending therefrom to a distal end 118. The catheter shaft 102 may be any standard construction catheter shaft, such as, but not limited to, standard percutaneous transluminal angioplasty (PTA) catheter shafts having multi-lumen or coaxial construction catheter shafts. The catheter shaft 102 may be coupled to the handle 110 by any suitable method, for example, and not by way of limitation, adhesives or mechanical connectors. A tapered nose cone or distal tip 120 may be coupled to the distal end 118 of the catheter shaft 102 as shown in FIG. 1. The catheter shaft 102 includes a proximal portion 122, a distal portion 124, and a deflection zone portion 126 disposed between the proximal portion 122 and the distal portion 124. In embodiments herein, the deflection zone portion 126 of the catheter shaft 102 is formed of materials such that the deflection zone portion 126 is more flexible than the proximal portion 122 and the distal portion 124. The catheter shaft 102 is configured to bend or deflect at the deflection zone portion 126, as illustrated in FIG. 5, when the catheter shaft 102 transitions from the delivery configuration to the deflected configuration. When the catheter shaft 102 is in the deflected configuration, the catheter shaft 102 is configured such that the side opening 104 is on an inside radius of the bend or deflection of the deflection zone portion 126 of the catheter shaft 102. The proximal and distal portions 122, 124 of the catheter shaft 102 may be made from any suitable material, such as, but not limited to melt flow polymers such as polyamides (PA), polyether block amides (PEBA), thermoplastic elastomers (PEBAX^{®}), and nylon. The deflection zone portion 126 of the catheter shaft 102 may be made from a melt flow polymer such as a thermoplastic elastomer (e.g. PEBAX^{®}) or a nylon with a hardness (Shore D Durometer) with a value below that of the proximal and distal portions 122, 124 of the catheter shaft 102 as to allow for appropriate deflection of that segment. In an example, the proximal and distal portions 122, 124 of the catheter shaft 102 may be constructed of a melt flow polymer (PEBAX^{®} or Nylon) with a hardness of 70D, and the deflection zone portion 126 may be constructed of a melt flow polymer (PEBAX^{®} or Nylon) with an appropriate length and a hardness of 40D to facilitate the appropriate bending shape.

The side opening 104 is disposed within the deflection zone portion 126 of the catheter shaft 102. The side opening 104 proximally extends from a proximal end 128 to a distal end 130. The side opening 104 extends radially inward and is configured to expose a portion of the electrosurgical wire 106 from the proximal end 128 to the distal end 130 of the side opening 104. In the embodiment shown, the side opening 104 may be generally rectangular in shape, but that is not meant to be limiting, and the side opening 104 may be any size and/or shape for exposing the electrosurgical wire 106 when in the deflected configuration, as explained below.

In the embodiment shown, the electrosurgical leaflet laceration device 100 includes a plurality of lumens, as shown in FIG. 2-3. In particular, in the embodiment shown, the electrosurgical leaflet laceration device 100 includes a guidewire lumen 132, an electrosurgical wire lumen 134, and an inflation lumen 172. In the embodiment shown, the guidewire lumen 132 extends through the entirety of the electrosurgical leaflet laceration device 100 from the proximal end 112 of the handle 110 to the distal end 118 of the catheter shaft 102 and through the distal tip 120. The guidewire lumen 132 is configured to slidably receive a guidewire for tracking the electrosurgical leaflet laceration device 100 through the vasculature. As used herein, the term "slidably" denotes back-and-forth movement in a longitudinal direction, along or generally parallel to a central longitudinal axis CLA of the electrosurgical leaflet laceration device 100. The electrosurgical wire lumen 134 distally extends from the proximal end 108 of the catheter shaft 102 to an anchor 138 at or near the distal end 118 of the catheter shaft 102. The electrosurgical wire lumen 134 is configured to slidably receive the electrosurgical wire 106. Further the electrosurgical wire lumen 134 intersects with the side opening 104 such that the side opening 104 opens into the electrosurgical wire lumen 134. The inflation lumen 172 has a proximal end 174 in fluid communication with a proximal inflation port 176 and a distal end 178 in fluid communication with a distal inflation port 180, as shown in FIG. 2. The distal inflation port 180 is in fluid communication with an interior of the balloon 170. The proximal inflation port 176 fluidly connects with a source of inflation fluid such that inflation fluid from the source of the inflation fluid enters the proximal inflation port 176 and is delivered through the inflation lumen 172 and the distal inflation port 180 into the interior of the balloon 170. As will be understood by one skilled in the art, the handle 110 provides a luer, hub, or other type of fitting at the proximal inflation port 176 that may be connected to the source of inflation fluid.

The balloon 170 includes an uninflated or delivery state, and an inflated or expanded state. FIGS. 1-2 show the balloon 170 in the uninflated state. The balloon 170 is configured to splay or open a perforation in a valve leaflet when the balloon 170 is in the inflated state. The balloon 170 has a proximal end 182 coupled to the catheter shaft 102 and a distal end 184 coupled to the catheter shaft 102 distal of the proximal end 182. The balloon 170 may be a standard construction, compliant, balloon constructed of any suitable material, such as, but not limited to, nylon, plastic, rubber, and polyurethane. The balloon 170 may have a diameter in a range from about 1mm to about 10mm and may be any length as required by the application.

The electrosurgical wire 106 includes a proximal or loop portion 140 and a distal portion 142. The loop portion 140 is disposed within the handle 110 and includes a proximal end 144 coupled to a connector 146 and a distal end 148 operatively coupled to the actuator 116 of the handle 110, as shown in FIGS. 2 and 4. The connector 146 permits connection of the electrosurgical wire 106 to a suitable power source such that the electrosurgical wire 106 may be energized to cut or lacerate tissue of a valve leaflet. As will be understood by those skilled in the art, energy may be applied to heat the electrosurgical wire 106 to a temperature sufficient to cut tissue abutting or in contact with a non-insulated or bare wire cutting segment 158 of the electrosurgical wire 106, as described below. In embodiments herein, the electrosurgical wire 106 may have any diameter suitable for the purposes described herein. Although described herein as having a loop portion 140 and a distal portion 142, the electrosurgical wire 106 may be a unitary (single piece) from the proximal end 144 of the loop portion 140 to a distal end 152 of the distal portion 142. However, in other embodiments, the loop portion 140 and the distal portion 142 may be separate pieces coupled together via welding or coupled together via the actuator 116. The loop portion 140 offers slack in the electrosurgical wire 106 such that the actuator 116 can be operated as described below. A proximal end of the distal portion 142 of the electrosurgical wire 106 is operatively coupled to the actuator 116 of the handle 110. The distal end 152 of the electrosurgical wire 106 (also the distal end of the distal portion 142) is coupled to the anchor 138. The electrosurgical wire 106 is configured to provide electrical connectivity from the connector 146 in the handle 110 to the distal end 152 of the distal portion 142 of the electrosurgical wire 106.

As shown in FIG. 4, the electrosurgical wire 106 includes an insulative cover 150 to prevent heating portions of the electrosurgical leaflet laceration device 100 that are not desired to be heated. Thus, in the embodiment shown, the insulative cover 160 is disposed over the electrosurgical wire 106 along the entire length thereof except for the non-insulated cutting segment 158 that is aligned with the side opening 104 of the catheter shaft 102 in the deflected configuration and the proximal end 144 of the electrosurgical wire 106 for connection to the connector 146. Thus, the insulative cover 160 includes a proximal portion 154 proximal of the non-insulated cutting segment 158 and a distal portion 156 distal of the non-insulated cutting segment 158.

In the embodiment shown, the anchor 138 is a circular wire coupled to the distal end 118 of the catheter shaft 102. However, this is not meant to be limiting, and the anchor 138 may be of any suitable shape for the purposes described herein. In another embodiment (not shown), the distal end 152 of the electrosurgical wire 106 may also extend past the anchor 138 and the distal end 152 may be coupled to the distal tip 120 such that the distal tip 120 may be energized by the electrosurgical wire 106 and may be utilized to puncture the valve leaflet. In another embodiment, the anchor 138 may be eliminated and the distal end 152 of the electrosurgical wire 106 may be coupled to the distal end 118 of the catheter shaft or the distal tip 120.

In embodiments herein, the electrosurgical wire 106 is configured as a pull wire to transition the electrosurgical leaflet laceration device 100 from the delivery configuration shown in FIG. 1 to the deflected configuration shown in FIG. 5A. In an embodiment, the actuator 116 of the handle 110 is actuated to proximally pull the electrosurgical wire 106 relative to the catheter shaft 102. With the distal end 152 of the electrosurgical wire 106 fixedly coupled to the anchor 138 which is fixedly coupled to the catheter shaft 102, the distal end 118 of the electrosurgical wire 106 does not move relative distal end 118 of the catheter shaft 102. Thus, proximal pulling of the electrosurgical wire 106 places the electrosurgical wire 106 in tension, and the catheter shaft 102 bends or deflects at the deflection zone portion 126, thereby transitioning and the electrosurgical leaflet laceration device 100 from the delivery configuration shown in FIG. 1 to the deflected configuration shown in FIG. 5A. The distal end 152 of the electrosurgical wire 106 may be coupled to the anchor 138 by methods such as, but not limited to adhesives, mechanical coupling, welding, or any other method suitable for the purposes described herein, or may be formed integral with the anchor 138.

With the electrosurgical leaflet laceration device 100 in the deflected configuration, the non-insulated cutting segment 158 of the electrosurgical wire 106 is aligned with the side opening 104 of the catheter shaft 102 such that the non-insulated cutting segment 158 is exposed outside of the catheter shaft 102. The electrosurgical wire 106 may be energized, such as by the connector 146 including an on/off switch or button, a remote on/off switch or button, or other devices to energize the electrosurgical wire 106. In another embodiment, shown in FIG. 5B, the actuator 116 and the connector 146 may include contacts 117/147 such that the electrosurgical wire 106 may be energized only when the actuator 116 has been actuated such that the contacts 117/147 touch each other, thereby ensuring that the electrosurgical wire 106 is not inadvertently energized unless the electrosurgical leaflet laceration device 100 is in the deflected configuration. The contacts 117/147 may be used instead of or in addition to another on/off button/switch. Further, while described as contacts between the actuator 116 and the connector 146, this is not meant to be limiting, and the contact required may be between the actuator 116 and another part of the electrosurgical leaflet laceration device 100 or between other parts of the electrosurgical laceration device 100 so as to ensure that the electrosurgical wire 106 is not energized unless the electrosurgical leaflet laceration device 100 is in the deflected configuration.

In embodiments herein, a diameter and a length of the balloon 170 in the inflated state, a stiffness of the electrosurgical wire 106, and a deflection angle of the electrosurgical leaflet laceration device 100 in the deflected configuration may be optimized to apply appropriate tension to the valve leaflet LF for optimal laceration of the valve leaflet LF.

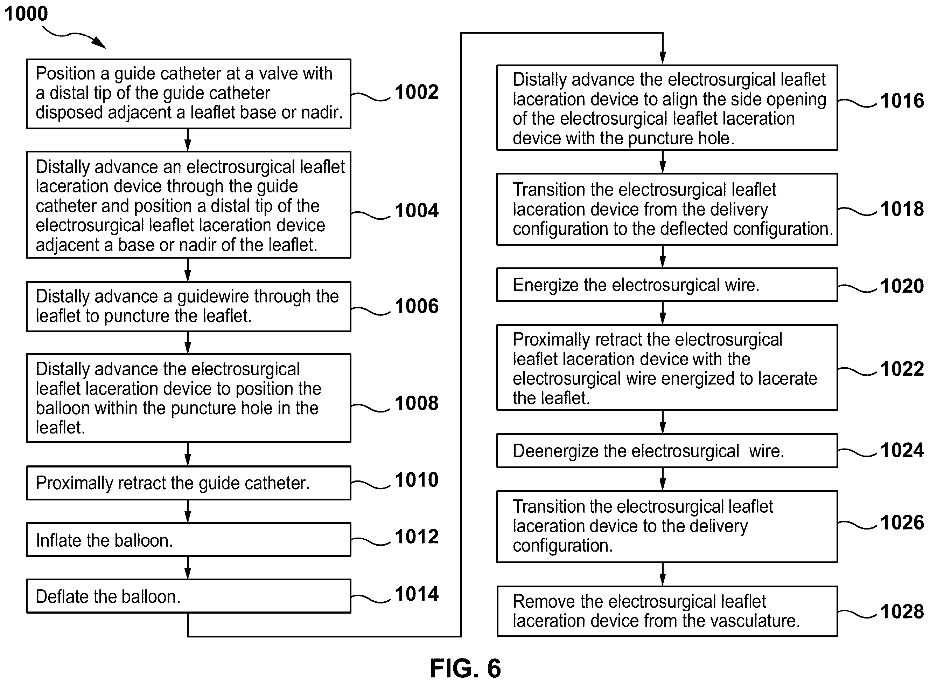

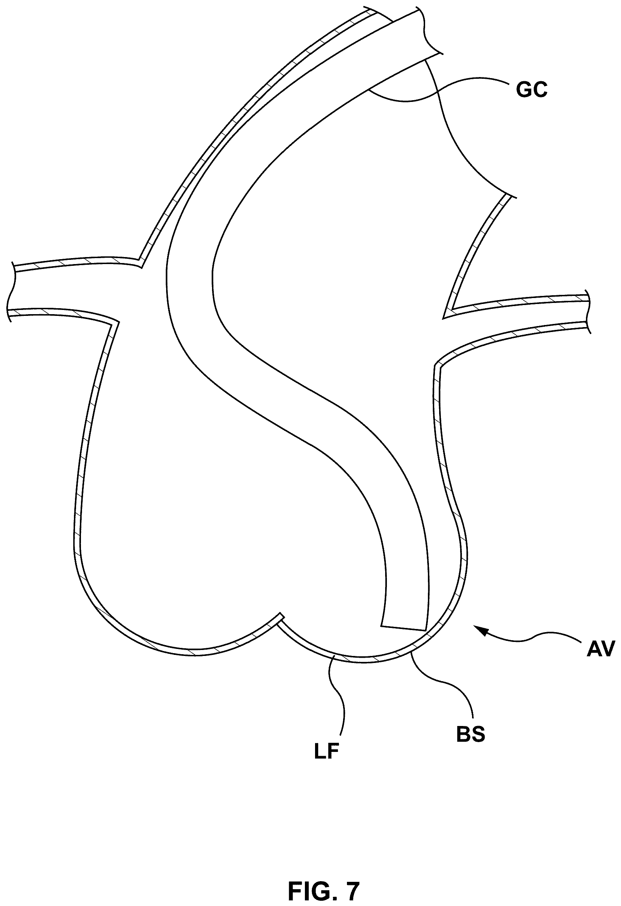

FIGS. 6-14 illustrate a method 1000 for lacerating a valve leaflet with an electrosurgical leaflet laceration device, in accordance with embodiments hereof. While described herein as lacerating a leaflet at an aortic valve of a heart, this is not meant to be limiting, and the method described herein may be utilized at other valve locations and/or to lacerate a prosthetic leaflet of a previously implanted heart valve prosthesis. FIG. 6 shows a flow chart with an overview of the method 1000 for lacerating a valve leaflet with the electrosurgical leaflet laceration device 100. FIGS. 7-14 illustrate steps of the method 1000 for lacerating a valve leaflet with the electrosurgical leaflet laceration device 100.

In a step 1002 of the method 1000, as shown in FIG. 7, a guide catheter GC is distally advanced through the vasculature of a patient and a distal end of the guide catheter GC is positioned adjacent a nadir or base BS of a valve leaflet LF to be lacerated. In the embodiment shown, the valve leaflet LF is a valve leaflet of a native aortic valve AV. However, as explained above, this is not meant to be limiting. The guide catheter GC may be introduced into the vasculature via a percutaneous entry point and advanced to the desired treatment location by established methods and procedures known to those skilled in the art.

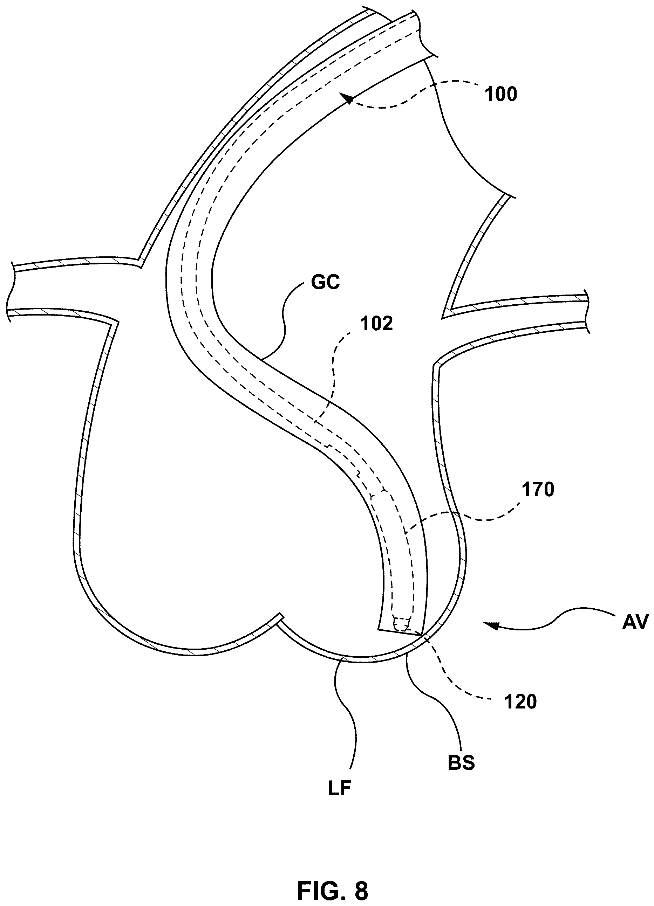

FIG. 8 shows a step 1004 wherein the electrosurgical leaflet laceration device 100 in the delivery configuration with the balloon 170 in the uninflated state is distally advanced within the guide catheter GC. The electrosurgical leaflet laceration device 100 is distally advanced such that the distal tip 120 of the catheter shaft 102 of the electrosurgical leaflet laceration device 100 is positioned adjacent to the base BS of the valve leaflet LF to be lacerated. In the embodiment shown in FIG. 8, the electrosurgical leaflet laceration device 100 is disposed on a downstream surface of the valve leaflet LF of the aortic valve AV.

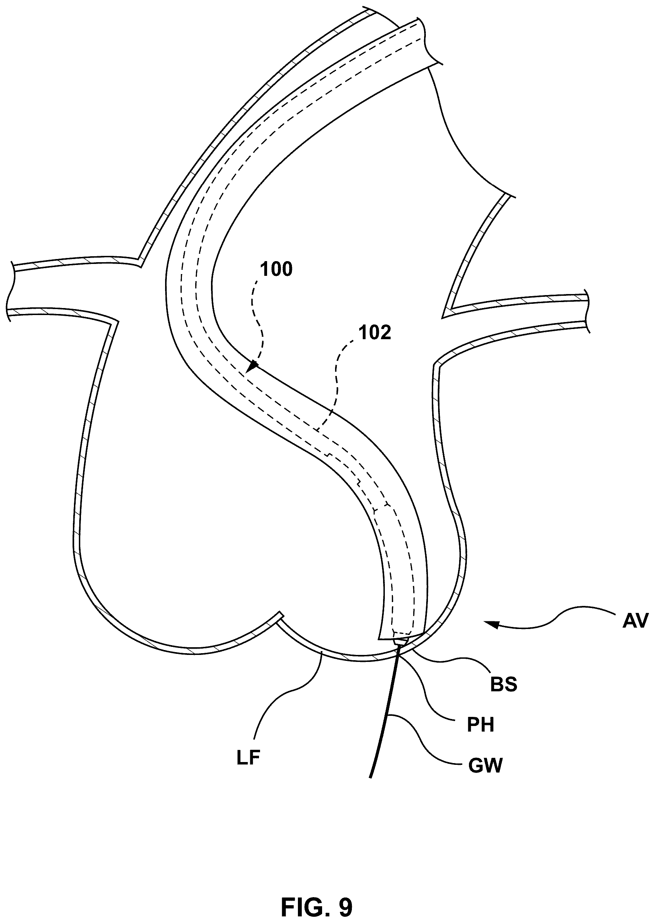

In a step 1006 of the method 1000, a guidewire GW is distally advanced through the guidewire lumen 132 of the catheter shaft 102 of the electrosurgical leaflet laceration device 100. The guidewire GW is distally advanced through the desired valve leaflet LF, thereby puncturing or perforating the tissue of the valve leaflet LF of the aortic valve AV adjacent to the base BS of the valve leaflet LF to form a puncture hole PH in the valve leaflet LF, as shown in FIG. 9.

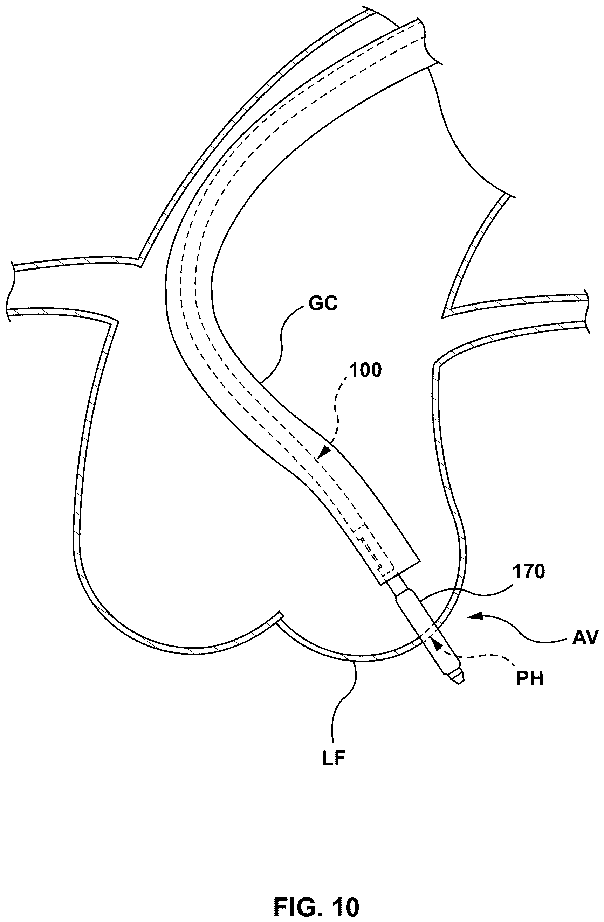

In a step 1008 of the method 100, the electrosurgical leaflet laceration device 100 is distally advanced over the guidewire GW and through the puncture hole PH in the valve leaflet LF of the aortic valve AV to position the balloon 170 of the electrosurgical leaflet laceration device 100 within the perforation PH in the valve leaflet LF, as shown in FIG. 10.

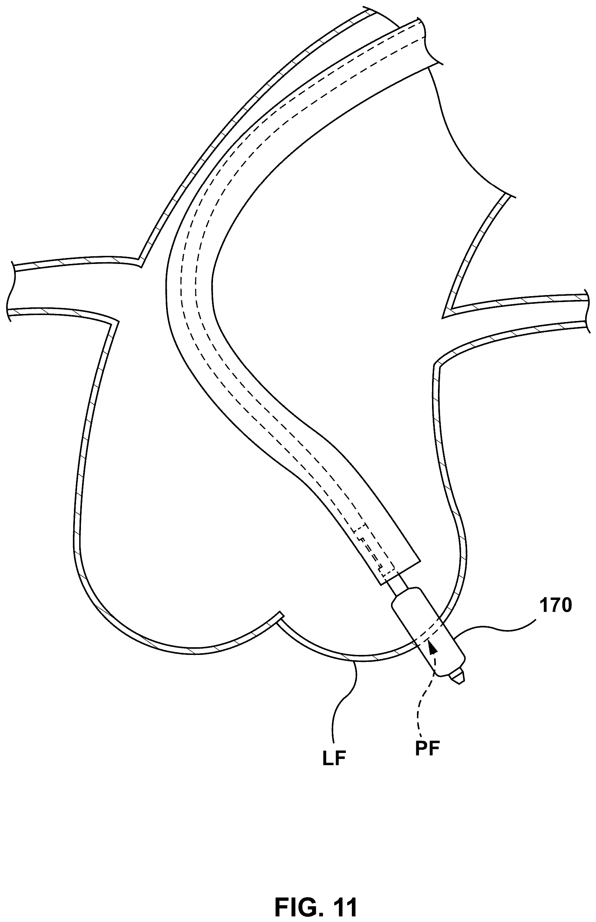

In a step 1010 of the method 1000, the guide catheter GC is proximally retracted to expose the balloon 170 from the guide catheter GC proximal of the valve leaflet LF to enable the balloon 170 to be inflated, as shown in FIG. 11.

As also shown in FIG. 11, in a step 1012 of the method 1000, inflation fluid is delivered to an interior of the balloon 170 to inflate the balloon 170 to transition the balloon 170 from the uninflated state to the inflated state. More specifically, and referring to FIG. 2, inflation fluid is delivered into the proximal inflation port 176, through the inflation lumen 172, and into the balloon 170 through the distal inflation port 180. Inflation of the balloon 170 radially expands the balloon 170 and, accordingly, radially expands or increases the size of the puncture hole PH in the valve leaflet LF. In an embodiment, the balloon 170 in the inflated state is large enough to create a larger splay at the base of the leaflet, but not so large to tear the leaflet in a direction not in line with the desired laceration direction.

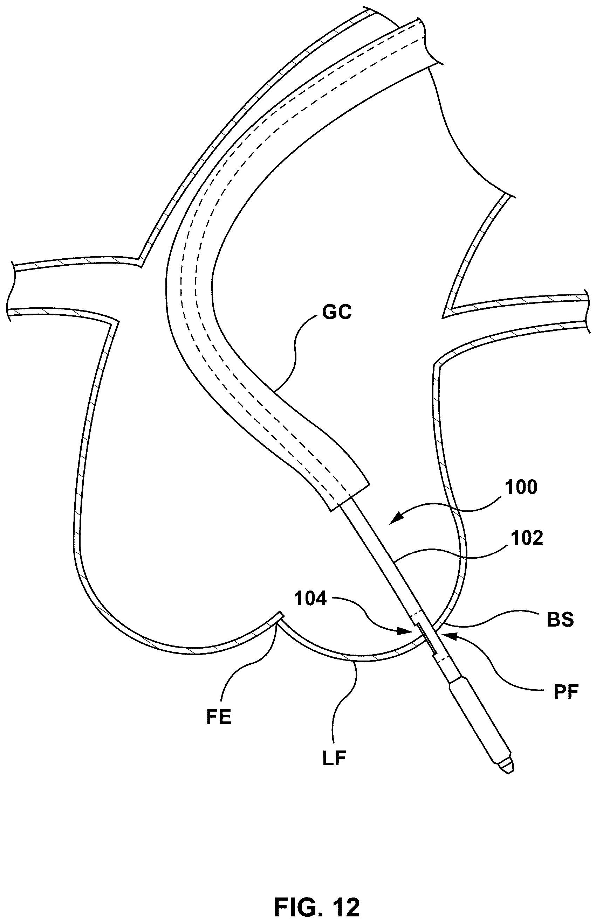

In a step 1014 of the method 1000, the inflation fluid is removed from the balloon 170 to deflate the balloon 170, i.e., transition the balloon 170 to the uninflated state. In a step 1016 of the method 1000, the electrosurgical leaflet laceration device 100 is distally advanced through the puncture hole PH in the valve leaflet LF to position the side opening 104 of the catheter shaft 102 within the puncture hole PH in the valve leaflet LF, as shown in FIG. 12. The side opening 104 of the catheter shaft 102 is rotationally oriented such that the side opening 104 is disposed towards the free edge FE of the leaflet LF, or away from the base BS of the valve leaflet LF.

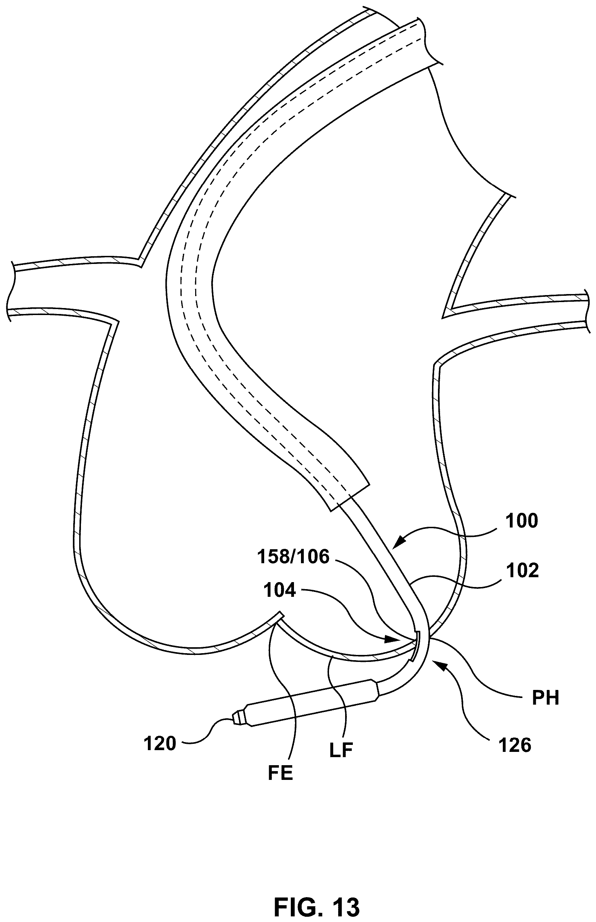

In a step 1018 of the method 1000, the actuator 116 of the handle 110 of the electrosurgical leaflet laceration device 100 is actuated to transition the electrosurgical leaflet laceration device 100 from the delivery configuration to the deflected configuration, as shown in FIG. 13. In particular, actuation of the actuator 116 applies a proximal force on the electrosurgical wire 106. Because the distal end 152 of the electrosurgical wire 106 is fixedly attached to the distal end 118 of the catheter shaft 102, such as via the anchor 138, the proximal force is transferred to the distal end 118 of the catheter shaft 102, thereby causing the catheter shaft 102 to bend or deflect at the deflection zone portion 126, thereby transitioning the electrosurgical leaflet laceration device 100 from the delivery configuration to the deflected configuration. Further, as explained above, as the electrosurgical wire 106 is proximally retracted to bend the catheter shaft 102, the cutting segment 158 of the electrosurgical wire 106 is longitudinally aligned with the side opening 104 of the catheter shaft 102 to expose the electrosurgical wire 106 to outside of the catheter shaft 102 through the side opening 104. Stated another way, as the electrosurgical leaflet laceration device 100 transitions to the deflected configuration, the cutting segment 158 of the electrosurgical wire 106 is exposed at the side opening 104 of the catheter shaft 102. When the electrosurgical leaflet laceration device 100 is in the deflected configuration, the distal tip 120 of the catheter shaft 102 is disposed near adjacent to the free edge FE of the valve leaflet LF and the cutting segment 158 of the electrosurgical wire 106 abuts or is contact with tissue of the valve leaflet LF within the puncture hole PH in the valve leaflet LF.

In a step 1020 of the method 1000, the electrosurgical wire 106 of the electrosurgical leaflet laceration device 100 in the deflected configuration is energized. In an embodiment, the electrosurgical wire 106 is energized with RF energy. The electrosurgical wire 106 may be energized via a power source (not shown) coupled to the connector 146. The energized electrosurgical wire 106 is heated to a temperature sufficient to cut tissue adjacent to the segment 158 of the electrosurgical wire 106.

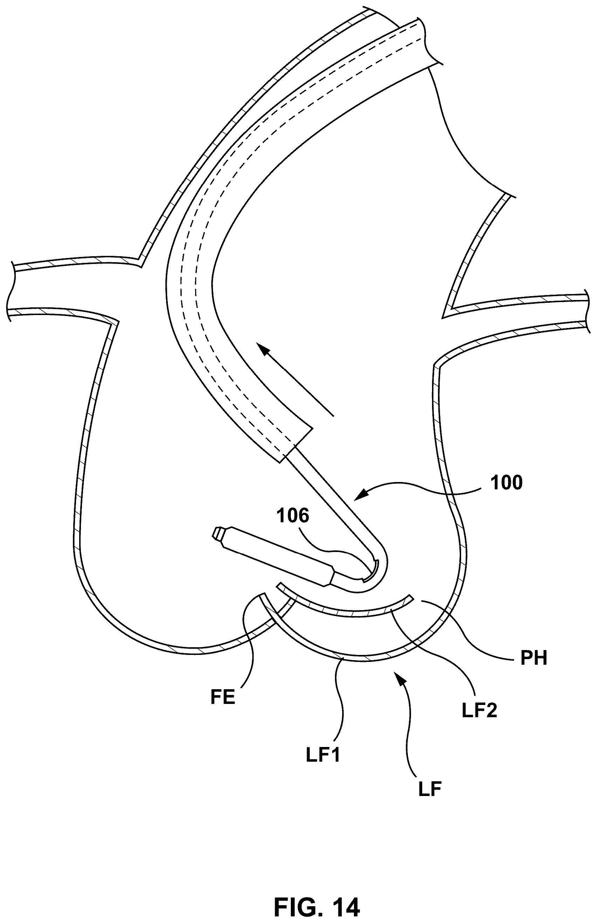

In a step 1022 of the method 1000, the electrosurgical leaflet laceration device 100 is proximally retracted such that the cutting segment 158 of the energized electrosurgical wire 106 cuts or lacerates the valve leaflet LF from the puncture hold PH to the free edge FE of the valve leaflet LF, as shown in FIG. 14. The valve leaflet LF is preferably lacerated or bisected into a first portion LF1 and a second portion LF2.

In a step 1024 of the method 1000, the electrosurgical wire 106 of the electrosurgical leaflet laceration device 100 is deenergized. In a step 1026 of the method 1000, the actuator 116 of the handle 110 of the electrosurgical leaflet laceration device 100 is de-actuated to release tension on the electrosurgical wire 106 to transition the electrosurgical leaflet laceration device 100 from the deflected configuration to the delivery configuration. In the embodiment of the actuator shown, this de-actuation is accomplished by distally advancing the actuator 116 to remove the proximal force on the electrosurgical wire 106.

In a step 1028 of the method 1000, the electrosurgical leaflet laceration device 100 is proximally retracted and removed from the vascular of the patient using established procedures. Those skilled in the art will recognize that other steps may be included in the method and/or steps may not be performed.

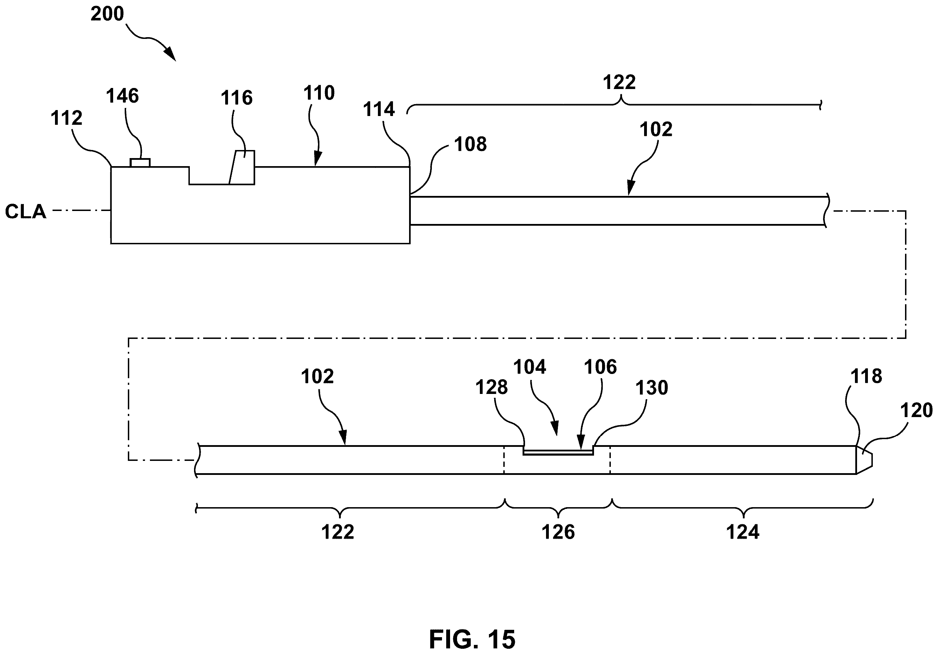

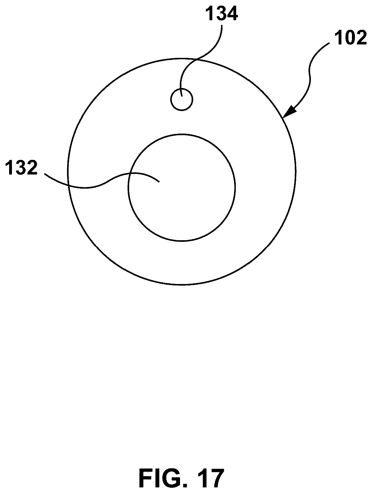

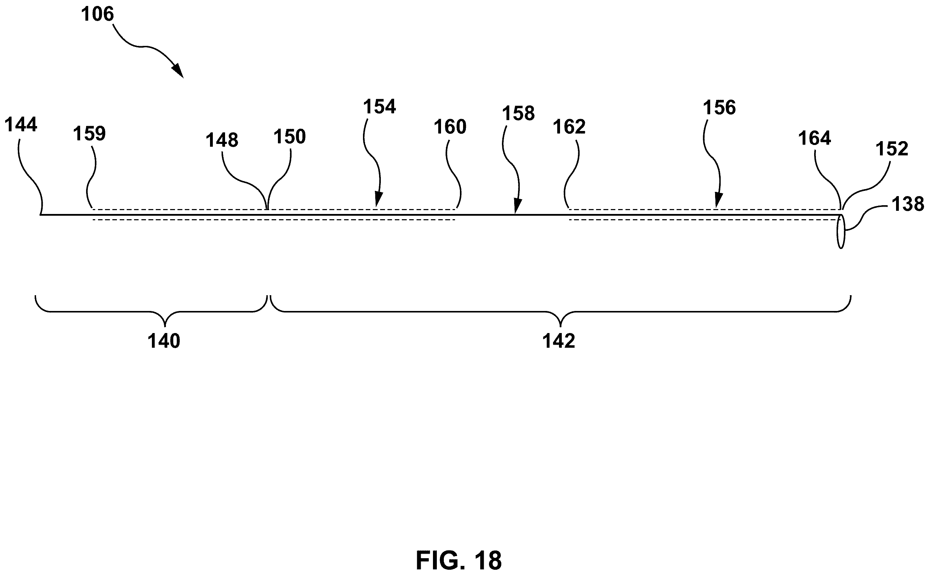

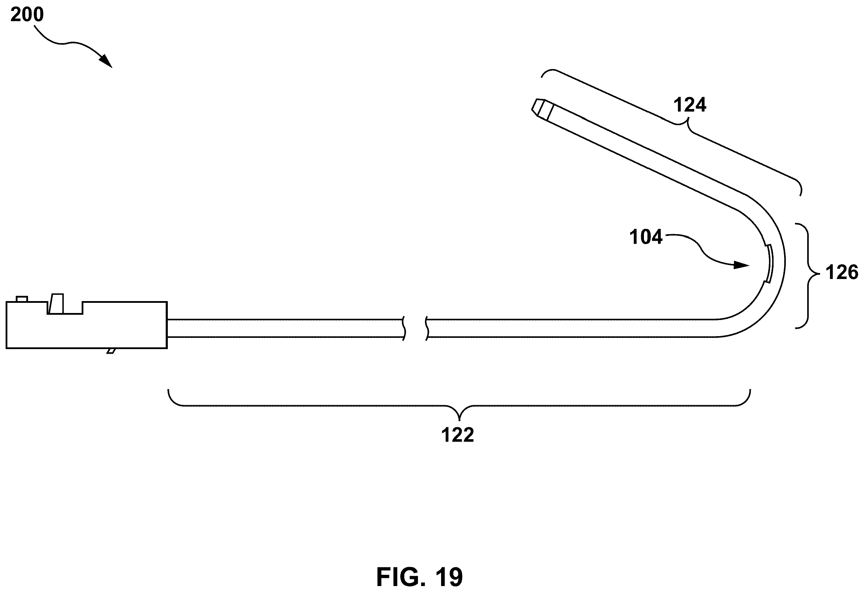

FIG. 15-19 illustrate an electrosurgical leaflet laceration device 200 according to embodiments hereof. The electrosurgical leaflet laceration device 200 is the same as the electrosurgical leaflet laceration device 100 shown in FIGS. 1-5B, except as described herein. Therefore, all of the details described above are incorporated into the description of FIGS. 15-19 and the same reference numerals are used. Thus, generally, as described above, the electrosurgical leaflet laceration device 200 includes a catheter shaft 102 having a side opening 104, an electrosurgical wire 106, and a handle 110. The electrosurgical leaflet laceration device 200 has a delivery configuration and a deflected configuration. However, the electrosurgical leaflet laceration device 200 differs from the electrosurgical leaflet laceration device 100 in that the electrosurgical leaflet laceration device 200 does not include a balloon on a distal portion of the catheter shaft 102 and does not include an inflation lumen or inflation ports.

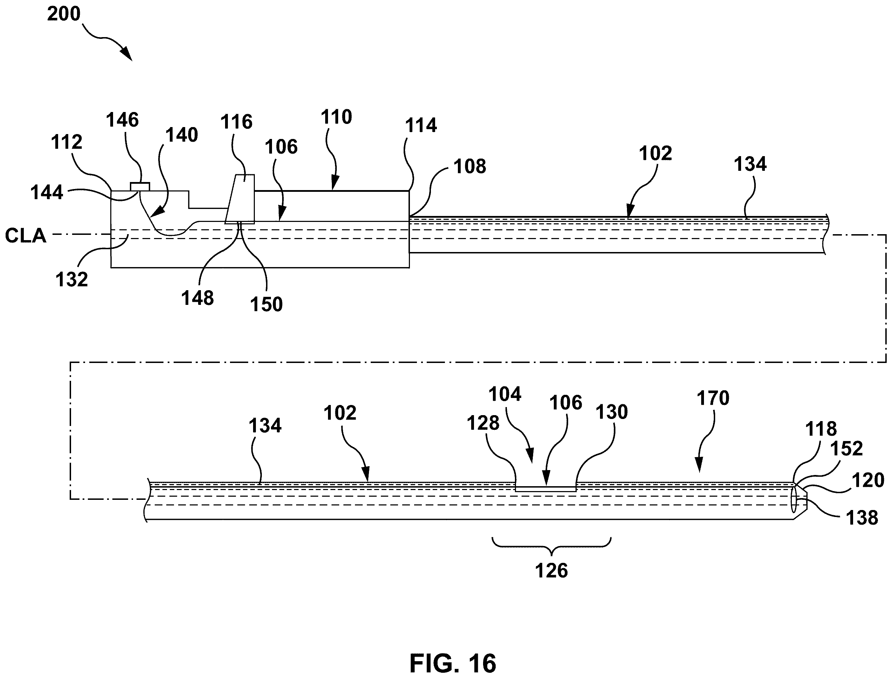

In embodiments herein, the electrosurgical leaflet laceration device 200 includes a guidewire lumen 132 and an electrosurgical wire lumen 134, as shown in FIGS. 16-17. The guidewire lumen 132 extends through the entirety of the electrosurgical leaflet laceration device from the proximal end 112 of the handle 110 to the distal end 118 of the catheter shaft 102 and extends through the distal tip 120. The guidewire lumen 132 is configured to slidably receive a guidewire. The electrosurgical wire lumen 134 extends from the proximal end 108 of the catheter shaft 102 to an anchor 138 at or near the distal end 118 of the catheter shaft 102. The electrosurgical wire lumen 134 is configured to slidably receive the electrosurgical wire 106.

The electrosurgical wire 106 includes a proximal or loop portion 140 and a distal portion 142, as described previously with respect to FIGS. 1-5B. Therefore, details of the electrosurgical wire 106 will not be repeated. The electrosurgical wire 106 is configured to provide electrical connectivity from the connector 146 in the handle 110 to the distal end 152 of the distal portion 142 of the electrosurgical wire 106. The electrosurgical wire 106 is further configured as a pull wire to transition the electrosurgical leaflet laceration device 200 from the delivery configuration to the deflected configuration. The electrosurgical wire 106 includes a non-insulated cutting segment 158 configured to cut or lacerate tissue abutting the non-insulated cutting segment 158 when the electrosurgical wire 106 is energized. The electrosurgical wire 106 is configured such that the non-insulated cutting segment 158 is exposed within the side opening 104 of the catheter shaft 102 when the electrosurgical leaflet laceration device 200 is in the deflected configuration.

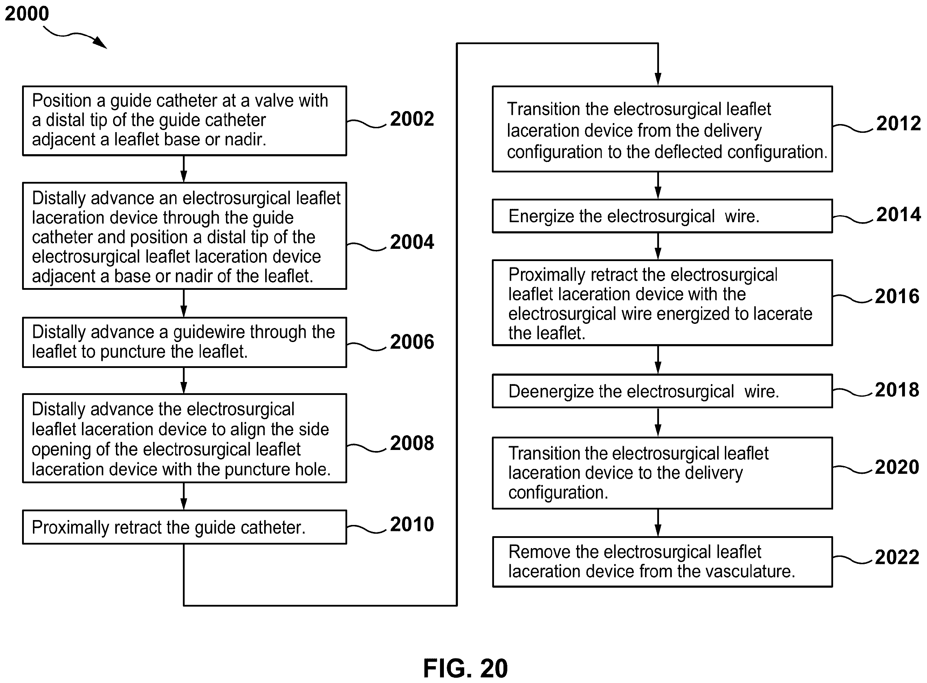

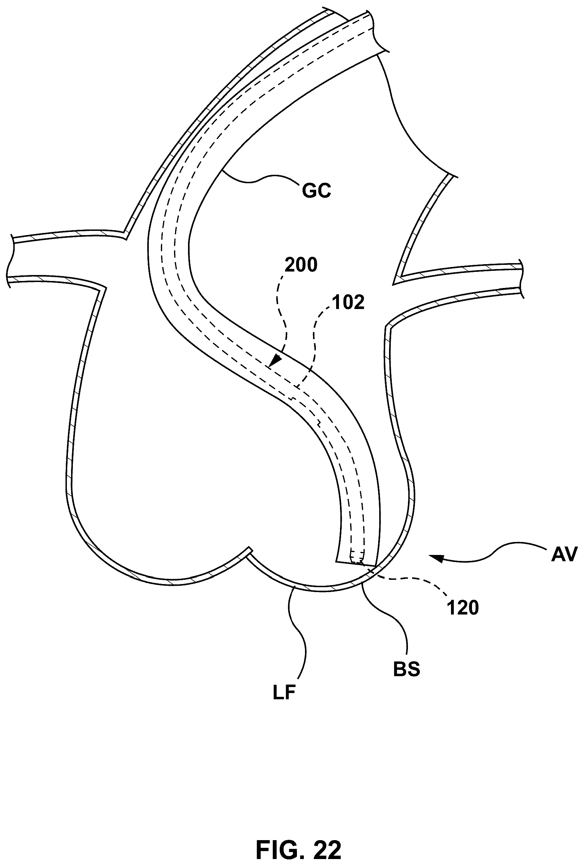

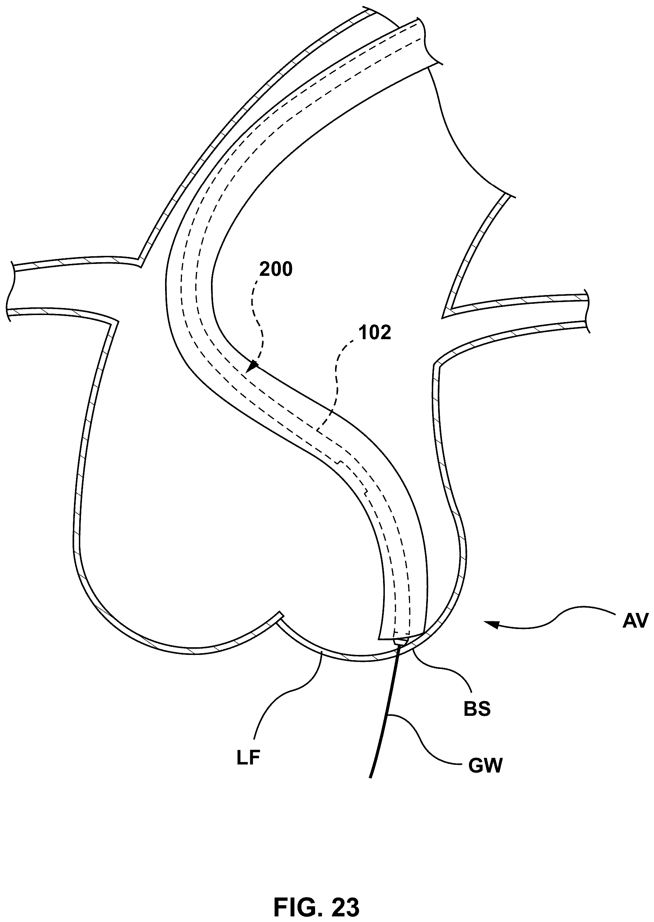

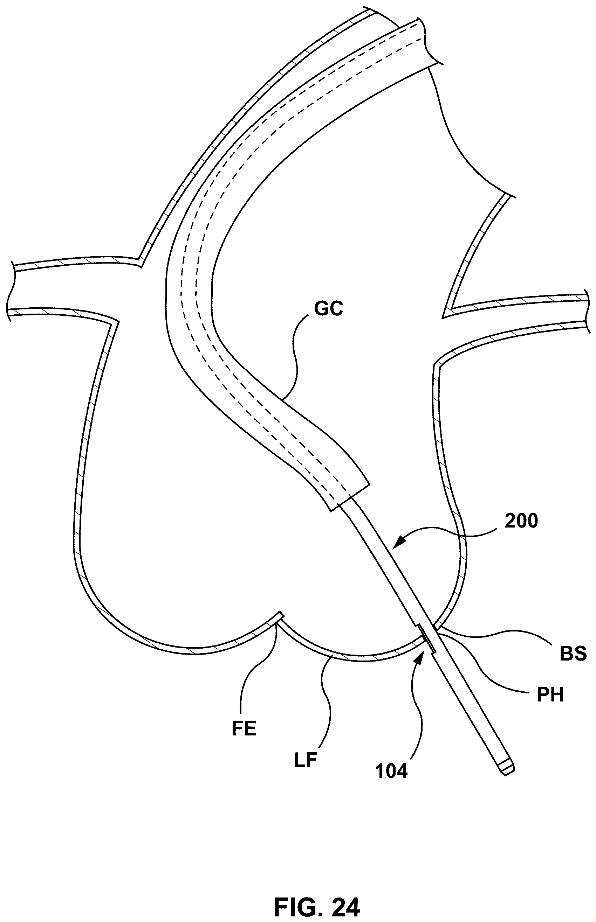

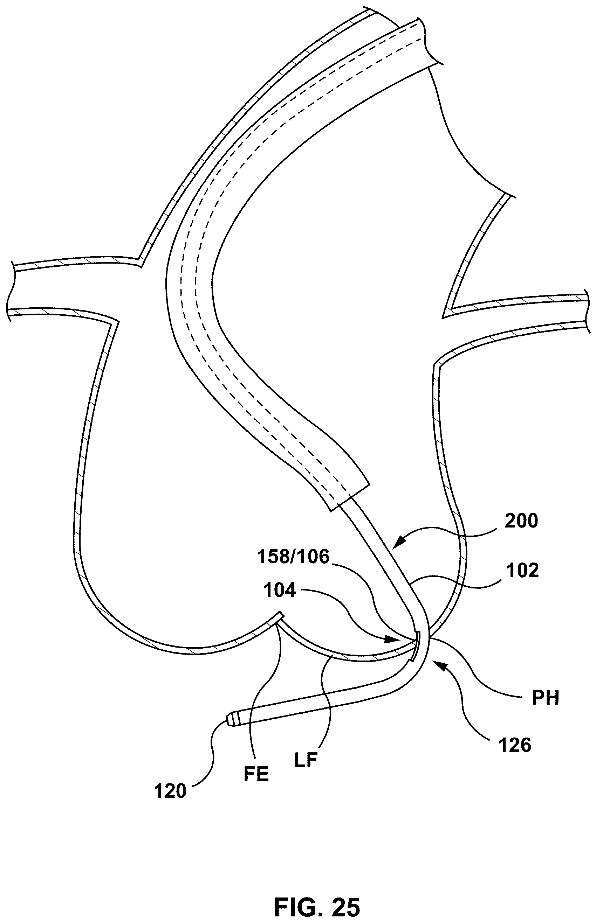

FIGS. 20-26 illustrate a method 2000 for lacerating a valve leaflet with an electrosurgical leaflet laceration device, such as the electrosurgical leaflet laceration device 200, in accordance with embodiments hereof. FIG. 20 shows a flow chart with an overview of the method 2000 for lacerating a valve leaflet with the electrosurgical leaflet laceration device 200. The method 2000 of FIG. 20 is similar to method 1000 of FIG. 6, and follows similar steps, except that the electrosurgical leaflet laceration device 200 does not include a balloon disposed on a distal portion of the catheter shaft 102. Therefore, the steps 1008, 1012, and 1014 for positioning, inflating, and deflating the balloon 170, respectively, of FIGS. 6-14 are omitted from the method 2000 of FIGS. 20-26. Due to the similarity of the methods, all of the details described above are incorporated into the description of FIGS. 20-26.

Accordingly, the method 2000 includes the step 2002 of positioning a guide catheter at a treatment site of a native heart valve, the step 2004 of advancing the electrosurgical leaflet laceration device 200 through the guide catheter to a position adjacent a base of a valve leaflet, and the step 2006 of distally advancing the guidewire to puncture the leaflet, as described above with respect to the steps 1002, 1004, 1006 of the method 1000.

In a step 2008 of the method 2000, the electrosurgical leaflet laceration device 200 is distally advanced through the puncture hole PH in the valve leaflet LF to position the side opening 104 of the catheter shaft 102 within the puncture hole PH in the valve leaflet LF. The side opening 104 of the catheter shaft 102 is rotationally oriented such that the side opening 104 is disposed towards a free edge FE of the valve leaflet LF. As briefly explained above, in an embodiment with the electrosurgical wire 106 also coupled to the distal tip 120 of the electrosurgical leaflet laceration device, the electrosurgical wire 106 may be energized prior to advancing or during advancing of the electrosurgical leaflet laceration device 200 through the puncture hole PH. In some embodiments, the energized distal tip 120 may be used to create the puncture hole PH.

In a step 2010 of the method 2000, the guide catheter GC is proximally retracted to enable transition of the electrosurgical leaflet laceration device 200 from the delivery configuration to the deflected configuration.

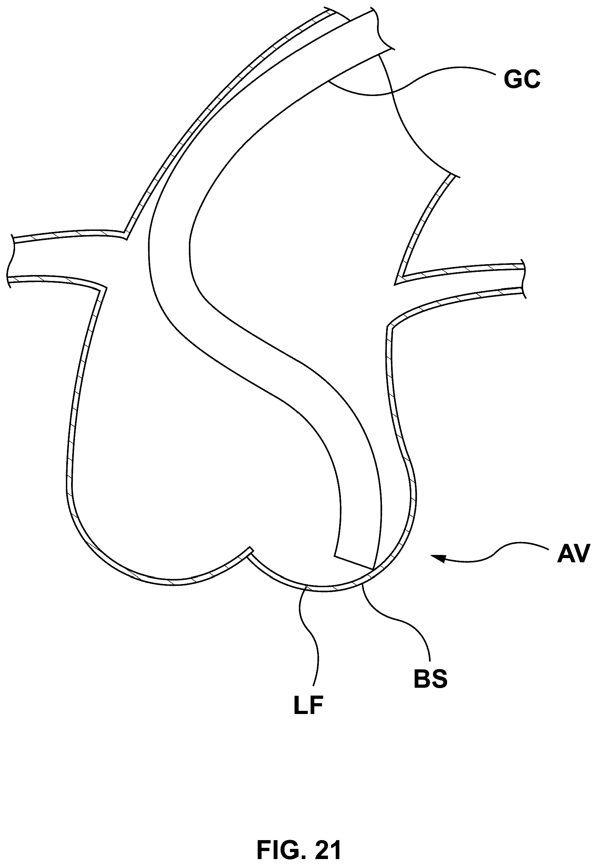

In a step 2012 of the method 200, the actuator 116 is actuated (e.g., proximally retracted in the embodiment shown) to transition the electrosurgical leaflet laceration device 200 from the delivery configuration to the deflected configuration, as shown in FIG. 21. Transition of the electrosurgical leaflet laceration device 100 to the deflected configuration also longitudinally aligns the cutting segment 158 of the electrosurgical wire 106 the side opening 104 of the catheter shaft 102 such that the cutting segment 158 abuts or contacts adjacent tissue of the valve leaflet LF.

In a step 2014 of the method 2000, the electrosurgical wire 106 of the electrosurgical leaflet laceration device 200 is energized, for example using RF energy. Energizing the electrosurgical wire 106 heat the electrosurgical wire 106 to a temperature sufficient to cut or splay tissue adjacent to the cutting segment 158 of the electrosurgical wire 106.

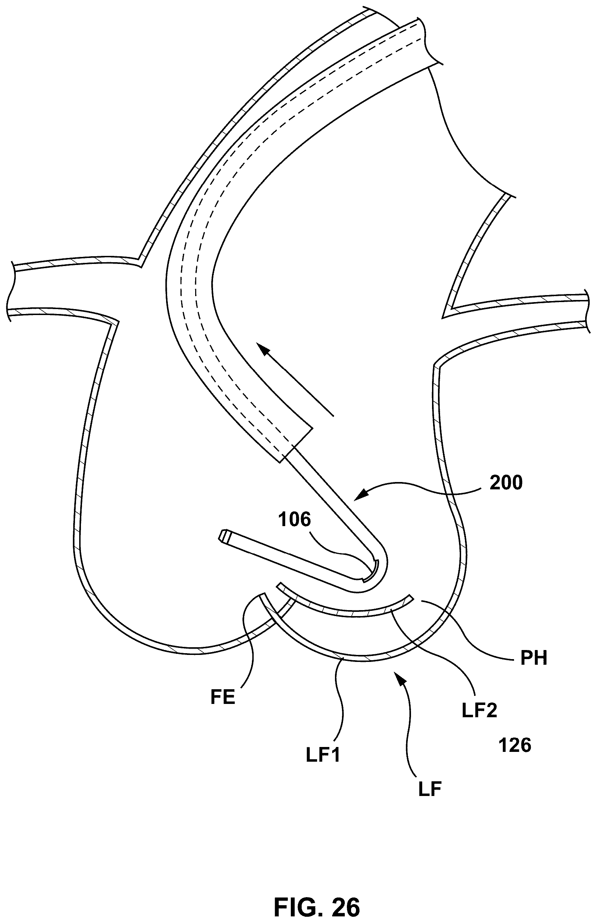

In a step 2016 of the method 2000, as shown in FIG. 26, with the electrosurgical wire 106 energized and the electrosurgical leaflet laceration device 200 in the deflected configuration such that the cutting segment is in contact with the valve leaflet LF, the electrosurgical leaflet laceration device 200 is proximally retracted such that the cutting segment 158 of the electrosurgical wire 106 cuts or lacerates adjacent tissue of the valve leaflet LF as the electrosurgical leaflet laceration device 200 is retracted. The valve leaflet LF is preferably bisected into a first portion LF1 and a second portion LF2.

Similar to the method described above, in steps 2018, 2020, and 2022 of the method 2000, the electrosurgical wire 106 of the electrosurgical leaflet laceration device 200 is deenergized, the proximal tension on the electrosurgical wire 106 is released such that the electrosurgical leaflet laceration device 200 transitions from the deflected configuration to the delivery configuration, and the electrosurgical leaflet laceration device 200 is proximally retracted to remove it from the vasculature of the patient.

It should be understood that, depending on the example, certain acts or events of any of the processes or methods described herein may be performed in a different sequence, may be added, merged, or left out altogether (e.g., all described acts or events may not be necessary to carry out the techniques).

While various embodiments have been described above, it should be understood that they have been presented only as illustrations and examples of the present technology, and not by way of limitation. It will be apparent to persons skilled in the relevant art that various changes in form and detail may be made therein without departing from the spirit and scope of the present technology. Thus, the breadth and scope of the present technology should not be limited by any of the above-described embodiments but should be defined only in accordance with the appended claims and their equivalents. It will also be understood that each feature of each embodiment discussed herein, and of each reference cited herein, may be used in combination with the features of any other embodiment. All patents and publications discussed herein are incorporated by reference herein in their entirety.

The following examples are illustrative of the techniques described herein.

Example 1. An electrosurgical leaflet laceration device having a delivery configuration and a deflected configuration, the electrosurgical leaflet laceration device comprising: a handle; a catheter shaft extending distally from the handle, the catheter shaft including an electrosurgical wire lumen and a side opening; and an electrosurgical wire disposed within the electrosurgical wire lumen of the catheter shaft, the electrosurgical wire having a cutting segment, wherein the side opening of the catheter shaft is configured to expose the cutting segment of the electrosurgical wire with the electrosurgical leaflet laceration device in the deflected configuration; and wherein the cutting segment of the electrosurgical wire is configured to be energized cut tissue abutting or in contact with the cutting segment.

Example 2. The electrosurgical leaflet laceration device of Example 1, wherein the electrosurgical wire is configured to transition the electrosurgical leaflet laceration device between the delivery configuration and the deflected configuration.

Example 3. The electrosurgical leaflet laceration device of Example 2, wherein a proximal portion of the electrosurgical wire is coupled to an actuator in the handle and a distal portion of the electrosurgical wire is fixedly coupled to a distal end of the catheter shaft, wherein the actuator is configured to apply or release tension on the electrosurgical wire to apply or release a proximal force on the distal end of the catheter shaft to bend the catheter shaft to transition the electrosurgical leaflet laceration device between the delivery configuration and the deflected configuration.

Example 4. The electrosurgical leaflet laceration device of Example 1, wherein the catheter shaft includes a proximal portion, a distal portion, and a deflection zone portion disposed between the proximal portion and the distal portion, wherein the deflection zone portion is more flexible than the proximal portion and the distal portion such that the catheter shaft is configured to bend or deflect at the deflection zone portion to transition the electrosurgical leaflet laceration device from the delivery configuration to the deflected configuration.

Example 5. The electrosurgical leaflet laceration device of Example 4, wherein the side opening is disposed within the deflection zone portion of the catheter shaft.

Example 6. The electrosurgical leaflet laceration device of Example 1, further comprising a balloon coupled to a distal portion of the catheter shaft, the balloon having an uninflated state and an inflated state, wherein the balloon is configured to splay or open a puncture hole in a valve leaflet with the balloon in the inflated state.

Example 7. The electrosurgical leaflet laceration device of Example 1, wherein the electrosurgical wire includes an insulative covering, and wherein the cutting segment is non-insulated.

Example 8. A method for lacerating a valve leaflet comprising: advancing an electrosurgical leaflet laceration device to a treatment site adjacent a valve leaflet; positioning a distal tip of a catheter shaft of the electrosurgical leaflet laceration device adjacent a base of the valve leaflet; distally advancing a guidewire through the catheter shaft and through the valve leaflet to puncture the valve leaflet to form a puncture hole; distally advancing the electrosurgical leaflet laceration device through the puncture hole in the valve leaflet; positioning a side opening of the catheter shaft within the puncture hole in the valve leaflet and adjacent to tissue of the valve leaflet; proximally retracting an electrosurgical wire of the electrosurgical leaflet laceration device to transition the electrosurgical leaflet laceration device from a delivery configuration to a deflected configuration in which the catheter shaft is bent adjacent the side opening and a cutting segment of the electrosurgical wire is longitudinally aligned with the side opening; energizing the electrosurgical wire; proximally retracting the electrosurgical leaflet laceration device in the deflected configuration such that the electrosurgical wire lacerates the valve leaflet; and deenergizing the electrosurgical wire.

Example 9. The method of Example 8, further comprising: after distally advancing the electrosurgical leaflet laceration device through the puncture hole in the valve leaflet and prior to positioning the side opening within the puncture hole, inflating a balloon of the electrosurgical leaflet laceration device to enlarge the puncture hole.

Example 10. The method of Example 8, further comprising: after deenergizing the electrosurgical wire, proximally retracting the electrosurgical leaflet laceration device to remove the electrosurgical leaflet laceration from the treatment site.

Example 11. The method of Example 8, wherein the valve leaflet is a native heart valve leaflet.

Example 12. The method of Example 11, wherein the native heart valve leaflet is a native aortic valve leaflet.

Example 13. The method of Example 8, wherein the valve leaflet is a prosthetic valve leaflet of a previously implanted heart valve prosthesis.

Example 14. The method of Example 8, wherein proximally retracting the electrosurgical wire comprises actuating an actuator of the electrosurgical leaflet laceration device to apply a proximal force on a proximal portion of the electrosurgical wire coupled to the actuator, wherein a distal portion of the electrosurgical wire is fixedly coupled to a distal portion of the catheter shaft such that the proximal force causes the catheter shaft to bend.

Example 15. The method of Example 14, wherein the actuator comprises a first contact and the electrosurgical leaflet laceration device further comprises a second contact, wherein the first contact and the second contact must be in contact to energize the electrosurgical wire.

## Claims

1. An electrosurgical leaflet laceration device (100, 200) having a delivery configuration and a deflected configuration, the electrosurgical leaflet laceration device comprising:
a handle (110);
a catheter shaft (102) extending distally from the handle, the catheter shaft including an electrosurgical wire lumen (134) and a side opening (104); and
an electrosurgical wire (106) disposed within the electrosurgical wire lumen of the catheter shaft, the electrosurgical wire having a cutting segment (158),
wherein the side opening of the catheter shaft is configured to expose the cutting segment of the electrosurgical wire with the electrosurgical leaflet laceration device in the deflected configuration; and
wherein the cutting segment of the electrosurgical wire is configured to be energized cut tissue abutting or in contact with the cutting segment.

2. The electrosurgical leaflet laceration device (100, 200) of claim 1, wherein the electrosurgical wire (106) is configured to transition the electrosurgical leaflet laceration device between the delivery configuration and the deflected configuration.

3. The electrosurgical leaflet (100, 200) laceration device of claim 2, wherein a proximal portion (140) of the electrosurgical wire (106) is coupled to an actuator (116) in the handle (110) and a distal portion (142) of the electrosurgical wire (106) is fixedly coupled to a distal end (118) of the catheter shaft (102), wherein the actuator is configured to apply or release tension on the electrosurgical wire to apply or release a proximal force on the distal end of the catheter shaft to bend the catheter shaft to transition the electrosurgical leaflet laceration device between the delivery configuration and the deflected configuration.

4. The electrosurgical leaflet laceration device (100, 200) of any one of claims 1 through 3, wherein the catheter shaft (102) includes a proximal portion (122), a distal portion (124), and a deflection zone portion (126) disposed between the proximal portion and the distal portion, wherein the deflection zone portion is more flexible than the proximal portion and the distal portion such that the catheter shaft is configured to bend or deflect at the deflection zone portion to transition the electrosurgical leaflet laceration device from the delivery configuration to the deflected configuration.

5. The electrosurgical leaflet laceration device (100, 200) of claim 4, wherein the side opening (104) is disposed within the deflection zone portion (126) of the catheter shaft (102).

6. The electrosurgical leaflet laceration device (100) of any one of claims 1 through 6, further comprising a balloon (170) coupled to a distal portion (124) of the catheter shaft (102), the balloon having an uninflated state and an inflated state, wherein the balloon is configured to splay or open a puncture hole in a valve leaflet with the balloon in the inflated state.

7. The electrosurgical leaflet laceration device (100, 200) of any one of claims 1 through 6, wherein the electrosurgical wire includes an insulative covering (150), and wherein the cutting segment (158) is non-insulated.
